(19) Europäisches Patentamt
European Patent Office
Office européen des brevets

(11) Veröffentlichungsnummer: **0 005 182**
**B1**

(12) **EUROPÄISCHE PATENTSCHRIFT**

(45) Veröffentlichungstag der Patentschrift: **29.07.81**

(51) Int. Cl.³: **A 61 K 7/44**

(21) Anmeldenummer: **79101050.7**

(22) Anmeldetag: **06.04.79**

(54) **Lichtschutzmittel.**

(30) Priorität: **18.04.78 DE 2816819**

(43) Veröffentlichungstag der Anmeldung:
**14.11.79 Patentblatt 79/23**

(45) Bekanntmachung des Hinweises auf die Patenterteilung:
**29.07.81 Patentblatt 81/30**

(84) Benannte Vertragsstaaten:
**BE CH DE FR GB IT NL SE**

(56) Entgegenhaltungen:
**DE - B - 1 087 902**
**GB - A - 1 020 614**
**FR - A - 1 368 808**

(73) Patentinhaber: **BAYER AG**
**Zentralbereich Patente, Marken und Lizenzen**
**D-5090 Leverkusen 1, Bayerwerk (DE)**

(72) Erfinder: **Preuss, Reinhard, Dr.**
**Oderstrasse 55**
**D-4150 Krefeld (DE)**
Erfinder: **Charlet, Egbert, Dr.**
**Ölbergweg 13**
**D-5064 Rösrath (DE)**
Erfinder: **Finkel, Peter, Ing. grad.**
**Isidor-Caro-Strasse 56**
**D-5000 Köln 80 (DE)**
Erfinder: **Rosenkranz, Hans Jürgen, Dr.**
**Heinrich-Kauert-Weg 9**
**D-4150 Krefeld (DE)**

**EP 0 005 182 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Die vorliegende Erfindung betrifft Mittel zum Schutz der Haut vor UV-Strahlung.

## Lichtschutzmittel

Die Sonnenstrahlung umfaßt unter anderem den Bereich der ultravioletten Strahlung, der im Hinblick auf die Wirkung auf die menschliche Haut in verschiedene Bereiche aufgeteilt werden kann:

a) UV-Strahlung des Wellenlängenbereiches von 285—320 nm (Nanometer) (UV-B-Bereich) verursacht bei normalhäutigen Menschen einen Sonnenbrand (Erythem) und aufgrund einer photoinduzierten Melanogenese eine anschließende Pigmentation (indirekte Pigmentierung).

b) UV-Strahlung des Wellenlängenbereiches von 320—400 nm (UV-A-Bereich) bewirkt eine rasche, schwach ausgebildete Braunfärbung (Direktpigmentierung). Dieser Pigmentation liegt eine Photooxydation bestimmter, in der Haut vorhandener Vorläufer des Melanins zugrunde.

Die üblichen Sonnenschutzmittel enthalten Stoffe, die die UV-B-Strahlung mehr oder weniger stark absorbieren. Für besonders lichtempfindliche Personen und um bei intensiverer Sonnenstrahlung die gesamte Belastung der Haut durch UV-Strahlung auf ein gesundes Maß zu beschränken, sind jedoch Sonnenschutzmittel erforderlich, die auch UV-A-Strahlung absorbierende Stoffe (UV-A-Filter) enthalten.

Über den Einsatz in kosmetischen Sonnenschutzmitteln hinaus sind UV-A-Filter in der Dermatologie für solche Präparate von Interesse, die zur Chemotherapie von chronischen Lichtschäden, der Psoriasis und von gewerblichen Fotodermatosen, wie sie beim Umgang mit Teer, Kohle und Pech auftreten können, eingesetzt werden können.

Darüber hinaus bestehen Zusammenhänge zwischen der Belastung der Haut durch UV-A-Strahlung und ihrer Alterung. Außerdem gilt es als gesichert, daß eine größere Belastung der Haut durch UV-A-Strahlung, insbesondere bei genetisch bedingter UV-Sensibilität, ein größeres Risiko an Hautkrebs zu erkranken bedeutet. Aus allen diesen Gründen erscheint es notwendig, Mittel zu entwickeln, die auch einen wirksamen Schutz gegen UV-A-Strahlung bieten.

Angestrebt wurde daher die Schaffung eines Lichtschutzmittels, insbesondere auf der Basis einer Öl-Wasser-Emulsion, das auch gegen UV-A-Strahlung einen wirksamen Schutz bietet. Dazu ist es erforderlich, einen öllöslichen UV-A-Filter bereitzustellen, der die folgenden Bedingungen erfüllt:

1) ein Absorptionsmaximum bei 340 nm;

2) eine hohe spezifische Extinktion $E_1^1$ (das bedeutet: Wirtschaftlichkeit, geringere Belastung der Rezeptur und geringeres toxikologisches Risiko, da weniger Substanz auf die Haut gelangt);

3) eine ausgezeichnete Öllöslichkeit (kein Ausfallen bei tieferen Temperaturen, z.B. beim Transport oder Verwendung beim Wintersport);

4) UV-Stabilität;

5) Oxydationsstabilität;

6) Thermostabilitat;

7) pH-Stabilität;

8) toxikologische Unbedenklichkeit;

9) kein ausgeprägter Eigengeruch;

10) keine deutliche Eigenfärbung;

11) möglichst eine Flüssigkeit (leichtes Einarbeiten in die Rezeptur);

12) in ausreichender Reinheit mit technisch üblichen Mitteln synthetisierbar.

UV-A-Filter für kosmetische Zwecke sind zwar prinzipiell nicht neu. Die bekannten UV-A-Filter erfüllen jedoch die oben genannten Bedingungen nicht in ausreichendem Maße, insbesondere nicht, was das Absorptionsmaximum, die Höhe der spezifischen Extinktion $E_1^1$ und die Öllöslichkeit anbetrifft.

Überraschenderweise wurde gefunden, daß 4-Methoxybenzylidencyanessigester der allgemeinen Formel (I)

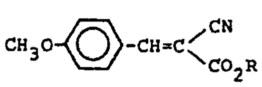

(I)

in der R den n-Hexyl-, n-Octyl- oder n-Decylrest oder einen primären Isoalkylrest mit 9 bis 10 Kohlenstoffatomen bedeutet, die oben genannten Bedingungen in optimaler Weise erfüllen.

Unter dem Rest R = primäres iso-Alkyl (Formel I) werden vorzugsweise solche Reste verstanden, die sich von den entsprechenden iso-Alkylalkoholen ableiten, welche über Hydroformylierung von Oligoolefinen und anschließender Hydrierung zugänglich sind. Bei den so erzeugten Alkoholen treten Isomerengemische auf, was bei dem entsprechenden Rest R ein entsprechendes oder im Zuge der Synthese verschobenes Isomerengemisch nach sich zieht.

Die Synthese der Verbindungen der Formel I erfolgt in Analogie zu literaturbekannten Verfahren (Knoevenagel-Kondensation) aus 4-Methoxybenzaldehyd und Cyanessigestern [z.B. Organikum, Ausg. 1967, S. 444].

Die entsprechenden Cyanessigester werden durch Umesterung eines gut zugänglichen Cyanessigesters, z.B. des Äthyl- oder Methylesters, mit dem entsprechenden Alkohol gewonnen.

Aus DT—PS 1 087 902 und US—PS 3 275 520 sind bereits Verbindungen der allgemeinen Formel (ii) bekannt.

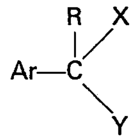

(II)

Wendet man die oben aufgestellten Bedingungen für einen UV-A-Filter auf diese breite Verbindungsklasse (II) an, so zeigt sich überraschenderweise, daß nur die wenigen erfindungsgemäßen Verbindungen der allgemeinen Formel (I) diese Bedingungen erfüllen:

1. *Ar. = aromatischer Rest:* Aus Gründen der Löslichkeit und um eine hohe spezifische Extinktion $E^1_1$ zu erzielen, muß dieser Rest möglichst klein sein. Kcmbiniert man diese Forderung mit derjenigen nach einem stabilen Molekül, so stellt der Phenylrest das Optimum dar. Heterocyclen wei z.B. Furan oder Pyrrol sind nicht ausreichend oxydationsstabil.

2. *Substituenten am aromatischen Rest:*

a) Stellung und Anzahl: ortho-Substitution bewirkt nur eine geringere Extinktion; meta-Substitution bewirkt zu kurzwellige Absorption; para-Substitution bewirkt hohe Extinktion und langwellige Absorption. Stehen mehrere Substituenten an einem Kern zur Diskussion, kommen nach den oben aufgeführten Gründen nur die in meta- und para-Stellung in Frage. Meta-para-disubstituierte Verbindungen zeigen aber eine niedrigere Extinktion, als die para-monosubstituierten. Die para-Monosubstitution stellt also das Optimum dar.

b) Art der Substituenten in para-Stellung; Halogen und Alkyl geben eine zu kurzwellige Absorption; Amino-und Alkylaminogruppen geben eine zu langwellige Absorption; eine Hydroxygruppe bewirkt eine nicht ausreichende Stabilität; O-Acylgruppen geben eine zu kurzwellige Absorption; eine Methoxygruppe bewirkt optimale Eigenschaften.

3. *Rest R (H, Alkyl, Aryl):* um eine optimale spezifische Extinktion zu erzielen, muß R gleich H sein.

Nach diesen Einschränkungen auf einen 4-Methoxyphenylrest und R = H, bleibt aus spektroskopischen Gründen für die Kombination X/Y nur noch Cyano-/Estergruppe bzw. Cyano//Alkylamidgruppe übrig. Die letztere ist aus Gründen der Löslichkeit und der spezifischen Extinktion $E^1_1$ der ersteren unterlegen, so daß das Optimum bei der erfindungsgemäßen Kombination Cyano-/Estergruppe liegt.

Diese Einflüsse gehen aus der nachstehenden Tabelle I deutlich hervor.

TABELLE I

| | Wellen-länge $\lambda$ max | molare Extinktion $\varepsilon$ | spez. Extinktion $E_1^1$ | Stabilität |
|---|---|---|---|---|
| 2-Methoxybenzylidencyanessigsäuremethylester | 354(297) | 10300(12100) | 470(560) | + |
| 3-Methoxybenzylidencyanessigsäuremethylester | 299 | 15300 | 710 | + |
| 3-Hydroxybenzylidencyanessigsäuremethylester | 303 | 18200 | 900 | – |
| 3-Hydroxybenzylidenmalonsäurediäthylester | 282 | 20300 | 770 | – |
| 4-Methoxybenzylidencyanessigsäuremethylester | 340 | 29300 | 1350 | + |
| 4-Methoxybenzylidenmalonsäurediisobutylester | 312 | 24800 | 740 | + |
| 4-Methoxybenzylidencyanessigsäureamid | 332 | 24300 | 1200 | + |
| 4-Methoxybenzylidencyanessigsäurediisobutylamid | 325 | 15900 | 510 | + |
| 4-Methoxybenzylidenacetessigsäureäthylester | 316 | 19000 | 770 | – |
| 4-Hydroxybenzylidencyanessigsäuremethylester | 348 | 27300 | 1350 | – |
| 4-Hydroxy-3-methoxybenzylidencyanessigsäuremethylester | 366 | 22400 | 960 | – |
| 4-Hydroxy-3-methoxybenzylidenmalonsäurediäthylester | 332 | 20750 | 630 | – |
| 4-Hydroxy-3-methoxybenzylidenacetessigsäureäthylester | 340 | 18200 | 690 | – |
| 3,4-Dimethoxybenzylidencyanessigsäuremethylester | 360 | 24000 | 970 | +– |
| 3,4-Dimethoxybenzylidenmalonsäurediäthylester | 308 | 20500 | 670 | +– |
| 4-Dimethylaminobenzylidencyanessigsäuremethylester | 421 | 19900 | 870 | – |
| 4-Dimethylaminobenzylidenmalonsäuredimethylester | 375 | 31400 | 1200 | – |
| 4-Dimethylaminobenzylidenmalonsäurediamid | 369 | 20400 | 870 | – |
| 4-Methacryloxybenzylidencyanessigsäuremethylester | 312 | 23000 | 1230 | – |

TABELLE I (Fortsetzung)

| | Wellenlänge $\lambda$ max | molare Extinktion $\varepsilon$ | spez. Extinktion $E^1_1$ | Stabilität |
|---|---|---|---|---|
| 4-Chlorbenzylidencyanessigsäuremethylester | 313 | 20900 | 940 | + |
| 3-Aminobenzylidencyansäuremethylester | 310 | 16800 | 830 | – |
| 4-Methylbenzylidencyanessigsäuremethylester | 317 | 25000 | 1230 | + |
| Furfurylidencyanessigsäuremethylester | 337 | 27000 | 1530 | – |
| α-Cyano-β-methyl-β-(-4-methoxyphenyl)-acrylsäuremethylester | 318 | 10900 | 460 | + |
| α-Cyano-β.β-diphenyl-acrylsäuremethylester | 298 | 14000 | 520 | + |

Nachdem somit gefunden wurde, daß 4-Methoxybenzylidencyanessigester bezüglich der spektralen Daten und der Stabilität optimal sind, konnte man annehmen, daß es nun relativ einfach sei, die geeignete Öllöslichkeit zu erzielen; denn normalerweise erhöht sich die Öllöslichkeit bei größeren organischen Molekülen, wenn sie mit einem aliphatischen Rest wachsender Kettenlänge verknüpft werden.

Überraschenderweise zeigte sich jedoch bei den erfindungsgemäßen Verbindungen eine sensible Abhängigkeit der Löslichkeit von der Kettenlänge und der Verzweigung des Restes R der Estergruppe, so daß schon Variationen von verzweigt zu unverzweigt bzw. Variationen von $C_n$ auf $C_{n+1}$ drastische Unterschiede der Löslichkeit herbeiführen.

Man kann also keineswegs einen beliebigen Alkylrest einsetzen, sondern ist auf die wenigen, ganz bestimmten bei der Formel I aufgeführten erfindungsgemäßen Alkylreste angewiesen.

Diese Abhängigkeiten kommen in der Tabelle II zum Ausdruck, aus der auch weitere hervorragende Eigenschaften der erfindungsgemäßen Verbindungen wie Schmelzpunkt und Extinktion deutlich werden.

Durchaus überraschend war es auch, daß die erfindungsgemäßen Verbindungen trotz ihres lipophilen Charakters, der eine Erleichterung der Penetration und Resorption durch die Haut zur Folge haben sollte, toxikologisch unbedenklich sind.

UV-A-Filter für kosmetische und dermatologische Lichtschutzmittel sind nicht neu. So werden z.B. 2,2'-Dihydroxy-4,4'-dimethoxy-benzophenon-5-sulfonsäure Natriumsalz (1), 2.2', 4,4'-Tetrahydroxy-benzophenon (2) und Dianisoylmethan (3) auf dem Markt angeboten.

Die Eigenschaften dieser bekannten Verbindungen werden denen der erfindungsgemäßen Derivate der Formel I in der Tabelle II gegenübergestellt. Aus diesem Vergleich gehen die überlegenen Eigenschaften der erfindungsgemäßen Verbindungen für den angestrebten Verwendungszweck hervor. Die bekannten Verbindungen (1—3) sind von der Löslichkeit her ungeeignet. (1) und (2) haben eine deutlich geringere spezifische Extinktion. (3) hingegen hat zwar eine hohe spezifische Extinktion, die aber zu langwellig ist, so daß der wichtige Bereich zwischen 320—400 mm nicht voll abgedeckt wird.

TABELLE II

| Erfindungsgemäße Verbindung der Formel I; R | Wellenlänge $\lambda$ max (nm) | molare Extinktion $\varepsilon$ $\frac{1000\ cm^2}{M}$ | Spez. Extinktion $E_1^1$ | Löslichkeiten bei RT (20—25°C) in Erdnußöl (%) | Erdnußöl/Paraffinöl (%) 1:9 | Schmelzpunkt (°C) |
|---|---|---|---|---|---|---|
| n-Butyl | 342 | 28300 | 1090 | 17 | 3 | 41—43 |
| n-Pentyl | 341 | 28600 | 1050 | 6—7 | 1,4 | 59—61 |
| n-Hexyl* | 342 | 28500 | 990 | 00 | 90 | Öl |
| n-Heptyl | 341 | 28300 | 940 | 33 | 7 | 34—36 |
| n-Octyl* | 341 | 30500 | 970 | 00 | 90 | 30 |
| n-Nonyl | 341 | 30000 | 910 | 55 | 10 | 32 |
| n-Decyl* | 341 | 27700 | 810 | 00 | 00 | Öl |
| n-Dodecyl | 342 | 28450 | 770 | 14 | —3 | 45—47 |
| iso-Hexyl | 342 | 26000 | 910 | 10 | —2 | 60—63 |
| iso-Octyl | 341 | 28500 | 910 | 45 | —7 | 30—31 |
| iso-Nonyl* | 341 | 28400 | 860 | 00 | 80 | Öl |
| iso-Decyl* | 341 | 27000 | 790 | 00 | 00 | Öl |
| Bekannte Verbindungen | | | | | | |
| (1) | 330 | 5920 | 160 | — | — | >350 |
| (2) | 351 | 15900 | 645 | 0,8 | — | 195 |
| (3) | 362 | 36000 | 1270 | 1 | — | 116.7 |

00 = unbegrenzt mischbar
— = praktisch unlöslich
* = erfindungsgemäße Verbindung

UV-Kinetiken ergaben eine ausgezeichnete UV-Stabilität der erfindungsgemäßen Verbindungen. Zur Verdeutlichung der Verträglichkeit möge folgender Hinweis dienen: die 14 Tage-LD 50 für den iso-Nonylester der Verbindung I beträgt 20.76 ml/kg, für den n-Hexylester 5.71 ml/kg. Eine Hautreizung bei Kaninchen konnte nicht festgestellt werden.

Gegenstand der Erfindung ist also ein Lichtschutzmittel, das in einer Öl-Phase mindestens eine der Verbindungen der Formel I als UV-A-Filter zum Schutz der Haut gegen UV-A-Strahlung neben anderen geigneten kosmetischen oder dermatologischen Zusätzen enthält. Das Lichtschutzmittel kann z.B. Anwendung finden als Schutz vor natürlicher Sonnenstrahlung, z.B. bei Freizeit oder gewerblich bedingtem Aufenthalt im Sonnenlicht, vor künstlichen Lichtquellen, z.B. Höhensonne und Solarium und zur Behandlum von pathogenen Sensibilitäten, z.B. genetische oder gewerbliche, gegenüber UV-A-Strahlung.

Im Einzelnen sind folgende öllösliche UV-A-Filter, für den erläuterten Verwendungszweck hervorragend geeignet:

4-Methoxy-benzyliden-cyanessigsäure-n-hexylester

4-Methoxy-benzyliden-cyanessigsäure-n-octylester

4-Methoxy-benzyliden-cyanessigsäure-n-decylester

4-Methoxy-benzyliden-cyanessigsäure-iso-nonylester

4-Methoxy-benzyliden-cyanessigsäure-iso-decylester

Der n-Hexyl- und der iso-Nonylester sind besonders hervorragend geeignet.

Diese Erkenntnisse sind besonders überraschend im Hinblick auf FR-A-1 368 808. Dort sind nämlich $\alpha$-Cyano-$\beta$,$\beta$-diphenylacrylester beschrieben, bei denen der zusätzliche Substituent am $\beta$-Kohlenstoffatom zu einer wesentlich geringeren spezifischen Extinktion, d.h. von 500 oder kleiner führt. Da außerdem die Absorptionsmaxima dieser Verbindungen bei 300 nm liegen, sind sie zwar als UV-B-Filter, nicht aber als UV-A-Filter geeignet. Die außerdem in der gleichen FR-Patentschrift genannten $\beta$,$\beta$-Diarylmethylen-malodinitrile zeigen zwar eine UV-A-Absorption, sind aber für den erfindungsgemäßen Zweck nicht geeignet, weil sie in pflanzlichen Olen, z.B. Erdnußöl, kaum löslich sind.

Die erfindungsgemäßen Mittel können in Form einer Flüssigkeit vorliegen, die aus einer einzigen Phase besteht und die ein einziges Lösungsmittel oder eine Mischung von Lösungsmittel, z.B. eine Oleo-Alkohol-Mischung enthält. Sie können auch als Dispersion, als Öl-in-Wasser- oder Wasser-in-Öl-Emulsion, als homogene Paste, als halbfestes Produkt oder als Produkt, das ein Treibmittel enthält vorliegen. Sie können Sonnenschutzmittel bilden, wie Öle, Lotionen, Aerosole (vom Typ Öl, Schaum oder "Spray"), sowie auch Creme für normale oder trockene Haut, Milch, Lippenstifte oder jegliche andere übliche kosmetische oder dermatologische Zubereitungen.

Als Bestandteile für die erfindungsgemäßen, oben näher definierten Mittel kann man insbesondere nennen: Lanolin, Vaseline, Triglyceride von Fettsäuren, Polyäthylenglykole, äthoxylierte Fettalkohole, Ester wie Isopropylpalmitat, Isopropylmystritat, Isopropylstearat, Oleyloleat, Butylstearat, tierische, pflanzliche, synthetische oder minerale Öle, Fettalkohole, niedere Alkohole, organische und mineralische Wachse. Diese Bestandteile werden in Mengen von etwa 1—97% Gew.—% verwendet.

Die erfindungsgemäßen Verbindungen der Formel I werden in den Lichtschutzmitteln gemäß der Erfindung in einer Konzentration von 0,2 bis 10 Gew.–%, bezogen auf das Gewicht der Zubereitung und vorzugsweise in 0,5 bis 6 %, verwendet, wobei der Rest der Zubereitung auf 100 Gew.–% ergänzt wird durch einerseits die üblichen kosmetischen bzw. dermatologischen Ingredienzien und andererseits das Lösungsmittel oder eine Mischung der Lösungsmittel, die in der gesamten Zubereitung enthalten sind.

Unter den kosmetischen und dermatologischen Zusätzen kann man Eindickungsmittel, reizlindernde und entzündungshemmende Mittel, Überfettungsmittel, Weichmacher, Benetzungsmittel, oberflächenaktive Mittel sowie Konservierungsmittel, Antischaummittel, Parfums bzw. Riechstoffe oder jeden anderen brauchbaren Zusatz nennen, wie er in der Kostmetik oder der Dematologie für den angestrebten Verwendungszweck üblich ist.

Die erfindungsgemäßen Mittel können farblos sein oder gefärbt sein mit solchen Farbstoffen und/oder Pigmenten, die gewöhnlich für Sonnenschutzmittel verwendet werden und insbesondere mit Eisenoxiden in Anteilen von etwa 0,001 bis 0,050 Gew.–%, bezogen auf das Gesamtgewicht der Zubereitung.

Enthält das erfindungsgemäße Mittel ein Treibmittel, so verwendet man insbesondere die Treibmittel auf Basis von Chlor-fluormethanen.

Die erfindungsgemäßen Zubereitungen können neben den UV-A-Filtern der Formel I auch UV-B-Filter enthalten wie:

p-Aminobenzoesäure
p-Aminobenzoesäureäthylester (25 Mol) äthoxyliert
p-Aminobenzoesäureäthylester (2 Mol) N-propoxyliert
p-Dimethylaminobenzoesäure-äthylester
p-Dimethylaminobenzoesäure-amylester
p-Dimethylaminobenzoesäure-2-äthylhexylester
p-Dimethylaminobenzoesäure-isoamylester
p-Dimethylaminobenzoesäure-hexylester
p-Dimethylaminobenzoesäure-heptylester

Salicylsäuremethylester
Salicylsäuredipropylenglykolester
Salicylsäure-homomenthylester
Salicylsäure-2-äthylhexylester
Triäthanolaminsalicylat

N-Acetylanthranilsäure-trimethyl-cyclohexylester
Anthranilsäurementhylester

Zimtsäurebenzylester
Zimtsäurementhylester und -homomenthylester
Zimtsäureoctylester
p-Isopropylzimtsäureäthylester
Diisopropylzimtsäureäthylester
Diisopropylzimtsäuremethylester
p-Methoxyzimtsäure-isoamylester

p-Methoxyzimtsäure-isopropylester
p-Methoxyzimtsäure-propylester

p-Methoxyzimtsäure-2-äthylhexylester
p-Methoxyzimtsäure-cyclohexylester
p-Methoxyzimtsäure und Salze
$\alpha$-Cyano-$\beta$-phenylzimtsäureäthylester
$\alpha$-Cyano-$\beta$-phenylzimtsäure-2-äthylhexylester

2-Hydroxy-4-methoxy-benzophenon
4-Phenyl-benzophenon
4-Phenyl-benzophenon-2-carbonsäure-2-äthylhexylester
2,2'-Dihydroxy-4-methoxy-benzophenon
2-Hydroxy-4-n-octyloxybenzophenon
2,2'-Dihydroxy-4,4'-dimethoxy-benzophenon
2-Hydroxy-4-methoxy-benzophenon-5-sulfonsäure
2,2'-Dihydroxy-4,4'-dimethoxy-benzophenon-5-sulfonsaures Natrium
2,4-Dihydroxy-benzophenon
2,2'-4,4'-Tetrahydroxy-benzophenon
2-Hydroxy-4-methoxy-4'-methyl-benzophenon

3-(4-Methylbenzyliden)-D.L-campher
3-(4-Methylbenzyliden)-D,L-campher-sulfonsaures Natrium
3-Benzyliden-D, L-campher

m-Digallussäuretrioleat
2-Phenylbenzmidazol-5-sulfonsäure
3,4-Dimethoxyphenol-glyoxylsaures Natrium

$\beta$-Imidazol-4(5)-acrylsäure (Urocaninsäure)
2-Phenyl-5-methyl-benzoxazol
Dibenzalazin
Dianisoylmethan
5-(3,3-Dimethyl-2-norbornyliden)-3-penten-2-on-2
(2'-Hydroxy-5'-methylphenyl)-benzotriazol
1-Phenyl-3-(3-pyridyl)-1,3-propandion

Besonders geeignete UV-B-Filter sind 2-Phenyl-5-methyl-benzoxazol, 2-Phenyl-benzimidazol-5-sulfonsäure und 4-Methoxy-zimtsäure-iso-amylester.

Beispiel 1: Sonnenschutzöl.

| | |
|---|---|
| p-Methoxy-benzyliden-cyanessigsäure-n-hexylester | 3% |
| Paraffinöl | 37% |
| Isopropylpalmitat | 60% |
| Parfümöl | q.s. |

Beispiel 2: Sonnenschutzöl.

| | |
|---|---|
| p-Methoxy-benzyliden-cyanessigsäure-n-octylester | 2% |
| p-Methoxy-zimtsäure-isoamylester | 2% |
| Erdnußöl | 46% |
| Paraffinöl | 50% |
| Parfümöl | q.s. |

Die Herstellung der Sonnenschutzöle erfolgt durch Mischen der aufgeführten Komponenten.

Beispiel 3: Lippenstifte.

| | |
|---|---|
| handelsübliche Lippenstiftmasse | 94% |
| p-Methoxy-benzyliden-cyanessigsäure-n-hexylester | 3% |
| p-Methoxyzimtsäure-isoamylester | 3% |

Die Lippenstiftmasse wird aufgeschmolzen und mit den beiden übrigen Komponenten gemischt. Die Masse wird in gekühlten Lippenstiftformen vergossen und die Formkörper nach dem Erkalten entnommen.

Beispiel 4: Sonnenschutz-Spray.

| | |
|---|---|
| Mischung nach Beispiel 1 | 40% |
| Treibgas-Mischung von Trichlorfluormethan und Dichlorfluormethan 70:30 | 60% |

Die beiden Komponenten werden in einen entsprechenden Druckgasbehälter gefüllt.

Beispiel 5: Sonnenschutz-Creme Typ O/W.

| | | |
|---|---|---|
| A. | -Fettalkohol-polyglykoläther auf der Basis von Stearyl- und Cetylalkohol | 7,5% |
| | -Entenbürzeldrüsenfett (künstlich) | 5,0% |
| | -Isopropylpalmitat | 6,0% |
| | -Caprylcaprinsäure-triglycerid | 11,0% |
| | -Cetylstearylalkohol | 2,0% |
| | -Silikonöl cP 100 | 0,5% |
| | p-Methoxy-benzyliden-cyanessigsäure-iso-nonylester | 2,0% |
| B. | -Wasser, entmineralisiert | 66,0% |
| | -Parfümöl | q.s. |
| | -Konservierungsmittel | q.s. |

Die Komponenten werden bei 70°C aufgeschmolzen und gemischt. Unter Rühren wird die Mischung portionsweise in das auf ca. 75°C erwärmte Wasser gegeben. Die entstandene Emulsion wird langsam und unter weiterem Rühren auf Raumtemperatur abgekühlt.

Beispiel 6: Sonnenschutz-Milch.

| | | |
|---|---|---|
| A. | -Kolloiddisperses Gemisch von Cetylstearyl-alkohol und Natrium-Cetylstearylsulfat mit einem nichtionogenen Emulgator | 3.15% |
| | -Decycloleat | 15.00% |
| | -4-Methoxy-benzyliden-cyanessigsäure-n-decylester | 2.00% |
| | (kann anstatt des n-Decylesters auch den iso-Decylester enthalten) | |
| B. | -Wasser | 79.85% |
| | -Parfüm | q.s. |
| | -Konservierungsmittel | q.s. |

Herstellung wie Beispiel 5.

**Patentansprüche**

1. Lichtschutzmittel, dadurch gekennzeichnet, daß sie 4-Methoxybenzylidencyanessigester der allgemeinen Formel (I)

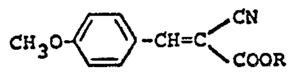

(I)

in der R den n-Hexyl-, n-Octyl- oder n-Decylrest oder einen primären Isoalkylrest mit 9—10 Kohlenstoffatomen bedeutet, enthalten.

2. Lichtschutzmittel nach Anspruch 1, dadurch gekennzeichnet, daß sie neben den 4-Methoxybenzylidencyanessigestern der allgemeinen Formel (I) übliche UV-B Filter und/oder bei Lichtschutzmitteln übliche Lösungsmittel und Zusatzstoffe enthalten.

3. Lichtschutzmittel nach Anspruch 1 und 2, dadurch gekennzeichnet, daß sie 4-Methoxybenzylidencyanessigsäure-n-hexylester enthalten.

4. Lichtschutzmittel nach Anspruch 1 und 2, dadurch gekennzeichnet, daß sie 4-Methoxybenzylidencyanessigsäure-iso-nonylester enthalten.

5. Lichtschutzmittel nach Anspruch 2, dadurch gekennzeichnet, daß sie als UV-B-Filter 2-Phenyl-5-methyl-benzoxazol, 2-Phenyl-benzimidazol-5-sulfonsäure oder 4-Methoxy-zimtsäure-isoamylester enthalten.

**Claims**

1. Agents for protection against light, characterised in that they contain 4-methoxybenzylidenecyanoacetic acid esters of the general formula (I)

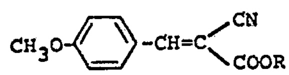

(I)

in which

R denotes the n-hexyl, n-octyl or n-decyl radical or a primary isoalkyl radical with 9—10 carbon atoms.

2. Agents for protection against light, according to Claim 1, characterised in that they contain customary UV-B filters and/or solvents and additives customary in agents for protection against light in addition to the 4-methoxybenzylidenecyanoacetic acid esters of the general formula (I).

3. Agents for protection against light, according to Claim 1 and 2, characterised in that they contain 4-methoxybenzylidenecyanoacetic acid n-hexyl ester.

4. Agents for protection against light, according to Claim 1 and 2, characterised in that they contain 4-methoxybenzylidenecyanoacetic acid iso-nonyl ester.

5. Agents for protection against light, according to Claim 2, characterised in that they contain 2-phenyl-5-methyl-benzoxazole, 2-phenyl-benzimidazole-5-sulphonic acid or 4-methoxy-cinnamic acid isoamyl ester as the UV-B filter.

**Revendications**

1. Agents de protection contre la lumière, caractérisé en ce qu'ils contiennent des esters d'acide 4-méthoxybenzylidènecyanacétique de formule générale

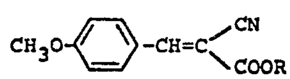

(I)

dans laquelle R représente un reste n-hexyle, n-octyle ou n-décyle ou un reste isoalkyle primaire en $C_9$ ou $C_{10}$.

2. Agents de protection contre la lumière, caractérisés en ce qu'ils contiennent, outre les esters d'acide 4-méthoxybenzylidènecyanacétique de formule générale (I), des filtres UV B habituels et/ou des solvants et additifs habituels dans des agents de protection contre la lumière.

3. Agents de protection contre la lumière selon les revendications 1 et 2, caractérisés en ce qu'ils contiennent le 4-méthoxybenzylidènecyanacétate de n-hexyle.

4. Agents de protection contre la lumière selon les revendications 1 et 2, caractérisés en ce qu'ils contiennent le 4-méthoxybenzylidènecyanacétate d'isononyle.

5. Agents de protection contre la lumière selon la revendication 2, caractérisés en ce qu'ils contiennent comme filtres UV B le 2-phényl-5-méthylbenzoxazole, l'acide 2-phényl-benzimidazole-5-sulfonique ou le 4-méthoxy-cinnamate d'isoamyle.